# EUROPEAN PATENT APPLICATION

(11) **EP 3 156 048 A1**
(43) Date of publication of application: **19.04.2017**
(21) Application number: 16193640.6
(22) Date of filing: 13.10.2016
(51) Int. Cl.: A61K 9/20, A61K 9/28, A61K 31/522

(54) **STABLE PHARMACEUTICAL COMPOSITION OF LINAGLIPTIN IN THE FORM OF IMMEDIATE RELEASE TABLETS**

(30) Priority: 13.10.2015 EP 15189539
(71) Applicant: Galenicum Health S.L., 08005 Barcelona (ES)
(72) Inventor: ARROYO HIDALGO, Sergio, 08005 Barcelona (ES)
(74) Representative: Galenicum Health S.L.

(57) **Abstract**

The present invention relates to stable pharmaceutical compositions of linagliptin in the form of immediate release tablets, to a process for the manufacture of said stable pharmaceutical compositions and to uniform pharmaceutical batches of said tablets.

## Description

The present invention relates to stable pharmaceutical compositions of linagliptin or a pharmaceutically acceptable salt thereof comprising a mixture of linagliptin in crystalline forms A and B, in the form of immediate release tablets, to a process for the manufacture of said stable pharmaceutical compositions and to uniform pharmaceutical batches of said immediate release tablets.

### STATE OF THE ART

Linagliptin, 8-[(3*R*)-3-aminopiperidin-1-yl]-7-but-2-ynyl-3-methyl-1- [(4-methylquinazolin-2-yl)methyl]purine-2,6-dione, has the following chemical structure:

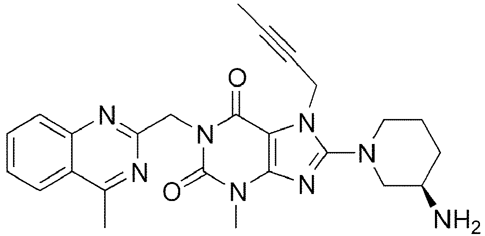

It is a dipeptidyl peptidase 4 (DPP-4) inhibitor developed by Boehringer Ingelheim for the treatment of type 2 diabetes mellitus. Type 2 diabetes mellitus affects more than 180 million people worldwide and particularly in the industrialised countries the incidence of the disorder is increasing. Type 2 diabetes mellitus is characterised by multiple metabolic abnormalities involving insulin resistance, impaired insulin secretion, and increased glucose production. Morbidity and mortality associated with type 2 diabetes mellitus is caused by macrovascular complications such as cardiovascular disease and microvascular complications such as retinopathy, neuropathy, and nephropathy.

Linagliptin is a highly soluble active substance. It is considered to be a Class III drug substance (high solubility, poor permeability) according to the Biopharmaceutical Classification System (BCS) due to incomplete oral systemic bioavailability (about 30% compared to intravenous administration) and the moderate permeability observed in Caco-2 cells. Due to the low active substance content in the final formulation, an adequate content uniformity may be ensured.

WO 2004/018468 and WO 2006/048427 describe the synthesis of linagliptin. Linagliptin crystalline forms A, B, C, D, and E are described in WO 2007/128721. According to WO 2007/128721, linagliptin prepared according to WO 2004/018468 is present at room temperature as a mixture of two enantiotropic polymorphs. The temperature at which the two polymorphs transform into one another is 25±15 °C. The two polymorphic forms do not differ with regard to biopharmaceutical properties. The pure high temperature form (polymorph A), can be obtained by heating the mixture to temperatures above 40 °C. The low temperature form (polymorph B) is obtained by cooling to temperatures under 10 °C. According to WO 2007/128721, the transition point between forms A and B is at room temperature, such that they exist as a polymorphic mixture.

Polymorphism, the occurrence of different crystal forms, is a property of some molecules and molecular complexes. A single molecule may give rise to a variety of polymorphs having distinct crystal structures and physical properties like melting point, thermal behaviors (e.g., measured by thermogravimetric analysis- "TGA", or differential scanning calorimetry- "DSC"), powder X-ray diffraction (XRD) pattern, infrared absorption fingerprint, and solid state NMR spectrum. One or more of these techniques may be used to distinguish different polymorphic forms of a compound.

US2012/0003313 discloses a pharmaceutical formulation comprising linagliptin, a first diluent, a second diluent, a binder, a disintegrant and a lubricant. The first diluent is mannitol, the second diluent is pregelatinized starch, the binder is copovidone, the disintegrant is corn starch, and the lubricant is magnesium stearate. It discloses the need of two diluents to obtain the desired formulations. Further, it specifies that excipients like microcrystalline cellulose and lactose were observed as being incompatible with DPP-4 inhibitors.

There is still a need in the art to provide an alternative pharmaceutical compositions of linagliptin having the desired *in vitro* and *in vivo* release properties, and in which mixtures of polymorphic forms are present.

Accordingly, it would be desirable to improve solubility of linagliptin in order to have good bioavailability of the drug. At the same time, being manufactured by a simple and robust process with high reproducibility and uniformity. Furthermore, it is desirable that the stability of the tablet is maintained and the dissolution profiles are good.

### DESCRIPTION OF THE INVENTION

The present invention provides stable pharmaceutical compositions of linagliptin or a pharmaceutically acceptable salt thereof in the form of immediate release tablets comprising a mixture of linagliptin crystalline forms A and B. Moreover, said compositions show a good stability. Surprisingly, said compositions have an improved solubility of the drug, resulting in good bioavailability and efficacy of the pharmaceutical composition.

The stable immediate release tablets as disclosed herein are uniform in content even when manufactured by wet granulation or direct compression. The pharmaceutical batches of the pharmaceutical compositions of the present invention present content uniformity even when the active ingredient is in low concentration (even less than 5 % of the total weight of the pharmaceutical composition). Moreover, the batches show good flowability.

It has been found that a specific particle size of the particles to be compressed to form the tablet cores, together with a specific amount of diluent, can be used to stabilize pharmaceutical compositions of linagliptin or a pharmaceutically acceptable salt thereof, especially immediate release tablets manufactured by wet granulation techniques or dry mix techniques, namely dry granulation or direct compression. The resulting compositions show an advantageous dissolution profile and/or good bioavailability and allows a high content uniformity and an effective production with regard to time and costs of pharmaceutical dosage forms.

In a first aspect, the present invention relates to a stable pharmaceutical composition of linagliptin in the form of immediate release tablets comprising a mixture of linagliptin crystalline forms A and B and a pharmaceutically acceptable excipient, wherein:
i) the total amount of active ingredient present in the pharmaceutical composition ranges from 1 to 10 % w/w and the total amount of diluent or mixture of diluents present in the pharmaceutical composition is at least 60 % w/w; both in respect of the total amount of the pharmaceutical composition, and
ii) the tablet consists of a core and a coating, wherein the core consists of compressed particles, wherein the D90 of such particles is less than 800 microns.

The term "pharmaceutical composition" as used herein refers to a finished dosage formulation, which contains the active agent linagliptin and which is in a form in which it can be marketed for use.

The term "active ingredient" or "active agent" refers to a therapeutically active compound, as well as any prodrugs thereof and pharmaceutically acceptable salts, hydrates and solvates of the compound and the prodrugs.

The term "pharmaceutically acceptable" indicates that the substance or composition must be compatible chemically and/or toxicologically, with the other ingredients comprising a formulation, and/or the mammal being treated therewith.

The term "compressed particles" as used herein refers to a mixture of particles or blend mixture before compression. In case of a direct compression it refers to a mixture of particles to be compressed into a tablet and in the case of a granulation it refers to a blend mixture comprising a granulate comprising intragranular components and mixture of particles comprising extragranular excipients.

The term "diluent" as used herein refers to pharmaceutically acceptable excipients which are added to the bulk volume of the active agent making up the solid composition. As a result, the size of the solid composition increases, which makes its size suitable for handling. Diluents are convenient when the dose of drug per solid composition is low and the solid composition would otherwise be too small. When the tablets are made by wet granulation, the diluent can be both intragranular and extragranular.

The term "stable" as used herein refers to a pharmaceutical composition comprising linagliptin wherein the total content of impurities originating from the decomposition of linagliptin does not exceed 5 % area, preferably 3 % area, more preferably 2 % area and most preferably 1 % area determined by liquid chromatography (HPLC) at 210 nm if such a composition is stored for 2 months at 40°C and 75 % relative humidity (RH).

An immediate release tablet as herein disclosed has to be understood as a tablet having a dissolution performance such as 60 % or more of the active agent contained in said pharmaceutical composition dissolves within 60 minutes (min). In a preferred embodiment, the immediate release composition as herein disclosed releases at least 80 % of the active agent in 60 minutes. In another embodiment, the pharmaceutical composition as herein disclosed releases at least 80 % of the active agent in 45 min, preferably in 35 min and more preferably in 30 min. In another embodiment, the pharmaceutical composition as herein disclosed releases at least 80 % of the active agent in 30 min and at least a 95 % of the active agent in 60 min. In a most preferred embodiment, the pharmaceutical composition as herein disclosed releases at least 80 % of the active agent in 15 min, and at least 95 % of the active agent in 60 min. The dissolution test for an immediate release pharmaceutical composition comprising the active agent as herein disclosed is performed in the following conditions: USP Apparatus: II (Paddles). Speed: 50 rpm. Medium: 0.1 N HCl pH 1.0. Wavelength 215 nm.

In a preferred embodiment of the first aspect, the D90 of the compressed particles is less than 500 microns. D90 represents the 90 percentile of the particle size distribution, as measured by the method of laser diffraction. Laser diffraction is a particle size measuring method which uses the average relative angular intensity of scattered light. Instruments that use laser light diffraction to measure particle size have been available for many years from different manufacturers (see e.g. US 5,610,712).

The particle size determination of the particles to be compressed is determined according to the following method: Apparatus: Malvern Mastersizer 2000S with sample dispersion unit.

In a preferred embodiment of the first aspect, the total amount of active ingredient present in the pharmaceutical composition ranges from 2 to 5 % w/w in respect of the total amount of the pharmaceutical composition.

In a preferred embodiment of the first aspect, the total amount of diluent present in the pharmaceutical composition ranges from 65 to 90 % w/w in respect of the total amount of the pharmaceutical composition.

In a preferred embodiment of the first aspect, the Carr's index of the particles to be compressed is less than 15. As used herein, "Carr's index" is based on the decrease in powder volume during tapping, and its usefulness lies in their ability to predict compressibility and flowability. The lower the number, the more free-flowing the powder. An increase in the value is proportional to adhesion and friction properties of a powder, including (attractive) triboelectric charge. The mathematical expression of each one is:

**Carr's Index (Compressibility Index) 100*(Vo -Vf)/ Vo)= 100"(Df -Do)/ Df**

| Angle of repose | Carr's Index | Fluidity |
|---|---|---|
| 25 - 30 | 0 - 10 | Excellent |
| 31 - 35 | 11 - 15 | Very good |
| 36 - 40 | 16 - 20 | Good |
| 41 - 45 | 21 - 25 | Tolerable |
| 46 - 55 | 26 - 31 | Poor |
| 56 - 65 | 32 - 37 | Very poor |
| > 66 | > 38 | Extremely poor |

Vo is the volume that a given mass of powder would occupy if let settled freely.
Vf is the volume of the same mass of powder would occupy after "tapping down".
Do is the density that a given mass of powder would occupy if let settled freely.
Df is the density that a given mass of powder would occupy after "tapping down".

In a preferred embodiment of the first aspect, the weight ratio linagliptin form A:form B ranges from 99:1 to 1:99, preferably the weight ratio linagliptin form A:form B ranges from 80:20 to 20:80, more preferably the weight ratio linagliptin form A:form B ranges from 70:30 to 30:70, even more preferably the weight ratio linagliptin form A:form B ranges from 70:30 to 40:60 and even yet more preferably the weight ratio linagliptin form A:form B ranges from 55:45 to 65:35.

In a preferred embodiment, the mixture of crystalline form linagliptin A and B used for the composition has a melting temperature of 198-202 °C.

In a preferred embodiment, the total content of impurities originating from the decomposition of linagliptin is less than 10 % w/w in respect of the total amount of the pharmaceutical composition after storage for 2 months at 40 °C at 75% RH, preferably the total content of impurities originating from the decomposition of linagliptin is less than 5 % w/w in respect of the total amount of the pharmaceutical composition after storage for 2 months at 40 °C at 75% RH, more preferably the total content of impurities originating from the decomposition of linagliptin is less than 3 % w/w in respect of the total amount of the pharmaceutical composition after storage for 2 months at 40 °C at 75% RH.

In a preferred embodiment, the total content of impurities originating from the decomposition of linagliptin is less than 10 % w/w in respect of the total amount of the pharmaceutical composition after storage for 2 months at 25°C at 60% RH, preferably the total content of impurities originating from the decomposition of linagliptin is less than 5 % w/w in respect of the total amount of the pharmaceutical composition after storage for 2 months at 25°C at 60% RH, more preferably the total content of impurities originating from the decomposition of linagliptin is less than 3 % w/w in respect of the total amount of the pharmaceutical composition for 2 months at 25°C at 60% RH.

In a preferred embodiment of the first aspect, the mixture of linagliptin crystalline forms A and B does not change into any other crystalline form.

In a preferred embodiment of the first aspect, the pharmaceutical composition is free of mannitol. In a more preferred embodiment, the pharmaceutical composition comprises only one diluent.

The prior art discloses the need of two diluents to obtain the desired formulations. However, the inventors have surprisingly found that linagliptin final dosage forms can be obtained with high quality using only one diluent.

In a preferred embodiment, the pharmaceutical composition comprises only one diluent different than mannitol.

In a preferred embodiment of the first aspect, the diluent is selected from of dicalcium phosphate, cellulose, compressible sugars, dibasic calcium phosphate dihydrate, lactose, trehalose, sorbitol, magnesium oxide, magnesium carbonate, microcrystalline cellulose, pregelatinized starch, tribasic calcium phosphate, and mixtures thereof; preferably the pharmaceutical composition comprises trehalose or pregelatinized starch or mixtures thereof.

In a preferred embodiment, the pharmaceutical composition comprises at least two diluents different than mannitol and pregelatinized starch. In a more preferred embodiment, the diluents are selected from of dicalcium phosphate, cellulose, compressible sugars, dibasic calcium phosphate dihydrate, lactose, trehalose, sorbitol, magnesium oxide, magnesium carbonate, microcrystalline cellulose, tribasic calcium phosphate, and mixtures thereof; preferably the pharmaceutical composition comprises trehalose, sorbitol, magnesium oxide or magnesium carbonate; more preferably the pharmaceutical composition comprises trehalose or sorbitol.

As used herein, "disintegrant" means a pharmaceutically acceptable excipient or a mixture of pharmaceutically acceptable excipients added to a formulation to facilitate its breakup or disintegration after administration. In a preferred embodiment of the first aspect, the total amount of disintegrant or disintegrants present in the pharmaceutical composition ranges from 1 to 15 % w/w in respect of the total amount of the pharmaceutical composition. Preferably, the pharmaceutical composition comprises at least a disintegrant selected from starch, pregelatinized starch, sodium starch glycolate, povidone, croscarmellose sodium, crospovidone, microcrystalline cellulose, sodium glycolate and mixtures thereof, more preferably the pharmaceutical composition comprises croscarmellose sodium, crospovidone, sodium glycolate or mixtures thereof.

The term "binder" as used herein is defined as an agent able to bind particles which cannot be bound only by a simple compression force. The binder may be present in the pharmaceutical composition in the form of a single compound or in the form of a mixture of compounds. In a preferred embodiment of the first aspect, the pharmaceutical composition comprises at least a binder, preferably the total amount of binder or binders present in the pharmaceutical composition ranges from 1 to 8 % w/w in respect of the total amount of the pharmaceutical composition. Preferably, the pharmaceutical composition comprises at least a binder selected from starch, maltodextrin, gelatin, copovidone, hydroxypropymethylcellulose, hydroxypropylcellulose, polyvinylpyrrolidone (PVP), carboxymethylcellulose or mixtures thereof. More preferably the pharmaceutical composition comprises copovidone.

As used herein, "lubricant" means a substance that reduces friction between the composition of the present invention and the surfaces of the apparatus used to compact the composition into a compressed form. The function of a lubricant in the product formulation is to prevent powder from sticking to the punches, dies, and other metal components of the tablet press. In a preferred embodiment of the first aspect, the pharmaceutical composition comprises at least a lubricant, preferably the total amount of lubricant or lubricants present in the pharmaceutical composition ranges from 0.5 to 5 % w/w in respect of the total amount of the pharmaceutical composition. Preferably, the pharmaceutical composition comprises at least a lubricant selected calcium stearate, magnesium stearate, polyethylene glycol, sodium stearyl fumarate, stearic acid, talc, or mixtures thereof. More preferably the pharmaceutical composition comprises magnesium stearate.

The inventors have surprisingly found that such quantities of lubricant help to avoid the tablet sticking during the process.

In a preferred embodiment of the first aspect, the pharmaceutical composition is manufactured by dry mix techniques or wet granulation techniques. In a preferred embodiment, the pharmaceutical composition is manufactured by dry mix techniques selected from dry granulation or direct compression, preferably direct compression.

In another preferred embodiment of the first aspect, the pharmaceutical composition is manufactured wet granulation techniques.

In a further embodiment of the pharmaceutical composition as herein disclosed, the pharmaceutical composition is prepared by direct compression or dry granulation.

As used herein, the term "granulate" refers to a population of granules. The granules as disclosed herein, can be prepared by any granulation method. As used herein, the term "granulation" refers to the process of agglomerating powder particles into larger agglomerates (i.e. granules) that contain the active agent. The term "granulation" includes dry and wet granulation techniques. The term "wet granulation" refers to any process comprising the steps of addition of a liquid to the powder starting materials, preferably kneading, and drying to yield a solid dosage form. The term "dry granulation" refers to any process that comprises compacting the powder, usually either by slugging or with a roller compactor, and preferably milling the compacted powder to obtain the granules. No liquid is employed for the dry granulation.

In another preferred embodiment of the first aspect, the D90 of the particles to be compressed is less than 500 microns, the total amount of the active ingredient present in the pharmaceutical composition ranges from 2 to 5 % w/w in respect of the total amount of the pharmaceutical composition, the weight ratio linagliptin form A:form B ranges from 70:30 to 30:70 and the pharmaceutical composition is free of mannitol.

In another preferred embodiment of the first aspect, the pharmaceutical composition comprises only one diluent selected from trehalose, pregelatinized starch and mixtures thereof, comprises at least a lubricant, preferably magnesium stearate wherein the total amount of lubricant or lubricants present in the pharmaceutical composition ranges from 0.5 to 5 % w/w in respect of the total amount of the pharmaceutical composition and the pharmaceutical composition is manufactured by dry mix techniques or wet granulation techniques, preferably direct compression.

In another preferred embodiment of the first aspect, the pharmaceutical composition comprises at least two diluents different than mannitol and pregelatinized starch, comprises at least a disintegrant selected from croscarmellose sodium, crospovidone, sodium glycolate and mixtures thereof and wherein the total amount of disintegrant or disintegrants present in the pharmaceutical composition ranges from 1 to 15 % w/w in respect of the total amount of the pharmaceutical composition and the pharmaceutical composition is manufactured by dry mix techniques or wet granulation techniques, preferably direct compression.

In a second aspect, the present invention relates to a pharmaceutical batch comprising at least 50,000 units of the pharmaceutical composition of the first aspect. In a preferred embodiment, the pharmaceutical batch comprises at least 100,000 units. In a more preferred embodiment, the pharmaceutical batch comprises at least 500,000 units.

In a preferred embodiment of the second aspect, the content of the active agent is uniform.

The term "batch" as used herein refers to a specific quantity of a drug or other material that is intended to have uniform character and quality, within specified limits, and is produced according to a single manufacturing order during the same cycle of manufacture. A batch, in the case of a drug product produced by continuous process, is a specific identified amount produced in a unit of time or quantity in a manner that assures its having uniform character and quality within specified limits (Code of Federal Regulations Title 21, Food and Drug Administration department of Health and Human Services, Subchapter C, Section 210.3 (b) (2) and (10)).

The term "pharmaceutical batch" as used herein refers to a batch as defined above of a pharmaceutical composition manufactured in accordance with the principles and guidelines of Good Manufacturing Practice (GMP) at an industrial scale and which is intended for commercialization (Directive 91/356/EEC).

The pharmaceutical composition may be manufactured at laboratory scale, not necessarily following GMP and not intended for commercialization. The pharmaceutical composition may also be manufactured for validation, following GMP. A batch of a pharmaceutical composition which is manufactured for validation is called "pilot batch".

Each pharmaceutical batch of finished product must fulfil the regulatory requirements of the corresponding Medicine Agency before being released for sale or supply, such as impurities thresholds and stability data.

The term "uniform" as used herein refers to the content of the active ingredient in the tablets of a pharmaceutical batch has to be homogeneous. According to the FDA criteria, uniformity is considered as achieving 90-110 % potency of the theoretical strength with a relative standard deviation (RSD) of less than 5 % for all samples (Guidance for Industry ANDA's: Blend Uniformity Analysis, published August 1999).

For the release of a pharmaceutical batch the distribution of the active ingredient in the tablets has to be homogeneous, that is, content uniformity is required. All batches are expected to be uniform within normal process variation. Process validation studies are conducted prior to the marketing of a drug product to assure that production processes are controlled. The test batch is manufactured prior to validation, yet it is the basis on which an application is approved (MANUAL OF POLICIES AND PROCEDURES, MAPP 5225.1).

It is essential, therefore, to assure that the test batch is uniform. In-process tests for uniformity should be conducted throughout the entire production process, e.g., at commencement or completion of significant phases (21 CFR 211.110). These tests should be designed to detect potential in-process anomalies (MAPP 5225.1).

In a preferred embodiment of the second aspect, the pharmaceutical compositions are packaged in a blister pack of aluminum/PVC, aluminum/aluminum or a perforated aluminium/PVC/polyvinyl acetate copolymer-acrylate blister. Although a blister aluminium/PVC is less protective than the aluminium/aluminium blister, the pharmaceutical composition as herein disclosed shows good stability in aluminum/PVC blisters.

The term "blister" or bubble pack refers to a sheet in a package construction with recesses designed to hold dosage forms. The sheet may be a plastic, a foil, or combination thereof. Normally, a blister is a product consisting of a flat structure in which blisters are formed, generally by means of a heating process, into which the single elements to be packaged are inserted. The blisters are then hermetically sealed using flat strips of appropriate thermomoldable materials (plastics, aluminum, paper), which represent the frangible element through which it is then possible to remove the product. The primary component of a blister pack is a cavity or pocket made from a formable web, usually a thermoformed plastic. This usually has a backing of paperboard or a lidding seal of aluminum foil or plastic. An aluminium/PVC blister refers to a blister the thermomoldable material is from PVC and the backing is a lidding seal of aluminium foil. Blister packs are commonly used as unit-dose packaging for pharmaceutical tablets, capsules or lozenges. Blister packs can provide barrier protection for shelf life requirements, and a degree of tamper resistance. In the USA, blister packs are mainly used for packing physician samples of drug products, or for Over The Counter (OTC) products in the pharmacy. In other parts of the world, blister packs are the main packaging type since pharmacy dispensing and re-packaging are not common. A series of blister cavities is sometimes called a blister card or blister strip as well as blister pack. The difference between a strip pack and blister pack is that a strip pack does not have thermo-formed or cold formed cavities; the strip pack is formed around the tablet at a time when it is dropped to the sealing area between sealing moulds. In some parts of the world the pharmaceutical blister pack is known as a Push-Through-Pack (PTP), an accurate description of two key properties (i) the lidding foil is brittle allowing to press the product out while breaking the lidding foil and (ii) a semi-rigid formed cavity being sufficiently collapsable to be able to dispense the tablet or capsule by means of pressing it out with your thumb. The main advantages of unit-dose blister packs over other methods of packing pharmaceutical products are the assurance of product/packaging integrity (including shelf life) of each individual dose and the possibility to create a compliance pack or calendar pack by printing the days of the week above each dose. Blister packs can be created by means of a form-fill-seal process at the pharmaceutical company or designated contract packer. A form-fill-seal process means that the blister pack is created from rolls of flat sheet or film, filled with the pharmaceutical product and closed (sealed) on the same equipment. Such equipment is called a blister line. There are two types of blister machine's design: rotary and flat-plate.

In a third aspect, the present invention relates to a process for preparing a pharmaceutical composition of the first aspect or the pharmaceutical batch of the second aspect, wherein the process comprises the following steps:
(i) weighing, optionally sieving and mixing the active agent and optionally at least one pharmaceutically acceptable excipient,
(ii) blending the mix of step (i),
(iii) optionally granulating the blend
(iv) optionally drying the granulate obtained in step (ii),
(v) compressing the blend obtained in step (ii) or the granules obtained in step (iii) or (iv) to form a tablet, and
(vi) coating the tablet composition.

In a fourth aspect, the present invention relates to the pharmaceutical composition or the pharmaceutical batch obtained by the process of the third aspect.

In a fifth aspect, the present invention relates to the pharmaceutical composition of the first or fourth aspect or the pharmaceutical batch of the second or fourth aspect, for use in the treatment of type 2 diabetes mellitus alone or in combination with other antidiabetic agents.

In a sixth aspect, the present invention relates to a blister pack comprising the pharmaceutical composition of the first or fourth aspect or the pharmaceutical batch of the second or fourth aspect, wherein said blister pack is preferably an aluminum/PVC blister, an aluminum/aluminum blister, or a perforated aluminium/PVC/polyvinyl acetate copolymer-acrylate blister.

In a seventh aspect, the present invention relates a cardboard box with a patient information leaflet comprising at least one aluminium/aluminium or aluminium/PVC blister pack of at least 4 units of the pharmaceutical composition of the first or fourth aspect or the pharmaceutical batch of the second or fourth aspect.

In a preferred embodiment, the pharmaceutical composition comprises from 4 to 10 mg of active agent per unit dose. Preferably, the pharmaceutical composition comprises 5 mg of active agent per unit dose. As used herein, the term "unit dose" or "unit dosage" refers to a physically discrete unit that contains a predetermined quantity of active ingredient calculated to produce a desired therapeutic effect. The unit dose or unit dosage may be in the form of a tablet, capsule, sachet, etc. referred to herein as a "unit dosage form".

In an eighth aspect, the present invention relates to a method for preparing a pharmaceutical dossier to obtain the marketing authorization of pharmaceutical composition of the first or third aspect comprising the following steps:
i) manufacturing at least one pharmaceutical batch of the second or fourth aspect;
ii) performing stability tests of the batches of step (i);
iii) compiling the results obtained in steps (i) to (iii); and
iv) providing the compiled results of step (iii) in a data carrier.

The term "pharmaceutical dossier" to obtain the marketing authorization refers to a dossier with data proving that the drug has quality, efficacy and safety properties suitable for the intended use, additional administrative documents, samples of finished product or related substances and reagents necessary to perform analyzes of finished product as described in that dossier.

In a ninth aspect, the present invention relates to a data carrier comprising the compiled results of a pharmaceutical dossier obtained by the method of the eighth aspect. In a preferred embodiment of the ninth aspect, said data carrier is selected from the group consisting of a digital data carrier such as CD, DVD, USB, hard drive; and paper.

The term "data carrier" refers to a device for recording (storing) information (data). The recording can be done using virtually any form of energy. A storage device may hold information, process information, or both. Most often the term is used with computers. Data carrier can permanently hold data, like files.

All percentages, parts, and ratios herein are by weight unless specifically noted otherwise. As used herein, the term "about" refers preferably to a range that is ±10 %, preferably ±5 %, or more preferably ±1 % of a value with which the term is associated.

Unless otherwise indicated, all the analysis methods are carried out according to the European Pharmacopoeia 7th edition.

Further aspects and embodiments of the invention are set out in the following numbered clauses:
1.- A stable pharmaceutical composition of linagliptin in the form of immediate release tablets comprising a mixture of linagliptin crystalline forms A and B and a pharmaceutically acceptable excipient, wherein:
   i) the total amount of active ingredient present in the pharmaceutical composition ranges from 1 to 10 % w/w and the total amount of diluent or mixture of diluents present in the pharmaceutical composition is at least 60 % w/w; both in respect of the total amount of the pharmaceutical composition, and
   ii) the tablet consists of a core and a coating, wherein the core consists of compressed particles wherein the D90 of such particles is less than 800 microns.
2.- The pharmaceutical composition according to the preceding clause, wherein the D90 of the particles to be compressed is less than 500 microns.
3.- The pharmaceutical composition according to any one of the preceding clauses, wherein the total amount of active ingredient present in the pharmaceutical composition ranges from 2 to 5 % w/w in respect of the total amount of the pharmaceutical composition.
4.- The pharmaceutical composition according to any one of the preceding clauses, wherein the total amount of diluent present in the pharmaceutical composition ranges from 65 to 90 % w/w in respect of the total amount of the pharmaceutical composition.
5.- The pharmaceutical composition according to any one of the preceding clauses, wherein the Carr's index of the particles to be compressed is less than 15.
6.- The pharmaceutical composition according to any one of the preceding clauses, wherein the weight ratio linagliptin form A:form B ranges from 99:1 to 1:99.
7.- The pharmaceutical composition according to any one of the preceding clauses, wherein the weight ratio linagliptin form A:form B ranges from 80:20 to 20:80.
8.- The pharmaceutical composition according to any one of the preceding clauses, wherein the weight ratio linagliptin form A:form B ranges from 70:30 to 30:70.
9.- The pharmaceutical composition according to any one of the preceding clauses, wherein the weight ratio linagliptin form A:form B ranges from 70:30 to 40:60.
10.- The pharmaceutical composition according to any one of the preceding clauses, wherein the weight ratio linagliptin form A:form B ranges from 55:45 to 65:35.
11.- The pharmaceutical composition according to any one of the preceding clauses, wherein the mixture of crystalline form linagliptin A and B used for the composition has a melting temperature of 198-202 °C.
12.- The pharmaceutical composition according to any one of the preceding clauses, wherein the total content of impurities originating from the decomposition of linagliptin is less than 10 % w/w in respect of the total amount of the pharmaceutical composition after storage for 2 months at 40 °C at 75% RH, preferably the total content of impurities originating from the decomposition of linagliptin is less than 5 % w/w in respect of the total amount of the pharmaceutical composition after storage for 2 months at 40 °C at 75% RH, more preferably the total content of impurities originating from the decomposition of linagliptin is less than 3 % w/w in respect of the total amount of the pharmaceutical composition after storage for 2 months at 40 °C at 75% RH.
13.- The pharmaceutical composition according to any one of the preceding clauses, wherein the total content of impurities originating from the decomposition of linagliptin is less than 10 % w/w in respect of the total amount of the pharmaceutical composition after storage for 2 months at 25°C at 60% RH, preferably the total content of impurities originating from the decomposition of linagliptin is less than 5 % w/w in respect of the total amount of the pharmaceutical composition after storage for 2 months at 25°C at 60% RH, more preferably the total content of impurities originating from the decomposition of linagliptin is less than 3 % w/w in respect of the total amount of the pharmaceutical composition for 2 months at 25°C at 60% RH.
14.- The pharmaceutical composition according to any one of the preceding clauses, wherein the mixture of linagliptin crystalline forms A and B does not change into any other crystalline form.
15.- The pharmaceutical composition according to any one of the preceding clauses, wherein the pharmaceutical composition is free of mannitol.
16.- The pharmaceutical composition according to any one of the preceding clauses, wherein the pharmaceutical composition comprises only one diluent.
17.- The pharmaceutical composition according to any one of the two clauses, wherein the diluent is selected from of dicalcium phosphate, cellulose, compressible sugars, dibasic calcium phosphate dihydrate, lactose, trehalose, sorbitol, magnesium oxide, magnesium carbonate, microcrystalline cellulose, pregelatinized starch, tribasic calcium phosphate, and mixtures thereof; preferably the pharmaceutical composition comprises trehalose or pregelatinized starch or mixtures thereof.
18.- The pharmaceutical composition according to any one of the clauses 1 to 15, wherein the pharmaceutical composition comprises at least two diluents different than mannitol and pregelatinized starch.
19.- The pharmaceutical composition according to the preceding clause, wherein the diluents are selected from of dicalcium phosphate, cellulose, compressible sugars, dibasic calcium phosphate dihydrate, lactose, trehalose, sorbitol, magnesium oxide, magnesium carbonate, microcrystalline cellulose, tribasic calcium phosphate, and mixtures thereof; preferably the pharmaceutical composition comprises trehalose, sorbitol, magnesium oxide, magnesium carbonate or mixtures thereof; more preferably the pharmaceutical composition comprises trehalose, sorbitol or mixtures thereof.
20.- The pharmaceutical composition according to any one of the preceding clauses, wherein the pharmaceutical composition comprises at least a disintegrant, preferably the total amount of disintegrant or disintegrants present in the pharmaceutical composition ranges from 1 to 15 % w/w in respect of the total amount of the pharmaceutical composition and/or the pharmaceutical composition comprises at least a disintegrant selected from starch, pregelatinized starch, sodium starch glycolate, povidone, croscarmellose sodium, crospovidone, microcrystalline cellulose, sodium glycolate and mixtures thereof, more preferably the pharmaceutical composition comprises croscarmellose sodium, crospovidone, sodium glycolate or mixtures thereof.
21.- The pharmaceutical composition according to any one of the preceding clauses, wherein the pharmaceutical composition comprises at least a binder, preferably the total amount of binder or binder present in the pharmaceutical composition ranges from 1 to 8 % w/w in respect of the total amount of the pharmaceutical composition and/or the pharmaceutical composition comprises at least a binder selected from starch, maltodextrin, gelatin, copovidone, hydroxypropymethylcellulose, hydroxypropylcellulose, polyvinylpyrrolidone (PVP), carboxymethylcellulose or mixtures thereof, more preferably the pharmaceutical composition comprises copovidone.
22.- The pharmaceutical composition according to any one of the preceding clauses, wherein the pharmaceutical composition comprises at least a lubricant, preferably the total amount of lubricant or lubricants present in the pharmaceutical composition ranges from 0.5 to 5 % w/w in respect of the total amount of the pharmaceutical composition and/or the pharmaceutical composition comprises at least a lubricant selected calcium stearate, magnesium stearate, polyethylene glycol, sodium stearyl fumarate, stearic acid, talc, or mixtures thereof, more preferably the pharmaceutical composition comprises magnesium stearate.
23.- The pharmaceutical composition according to any one of the preceding clauses, wherein the pharmaceutical composition is manufactured by dry mix techniques or wet granulation techniques.
24.- The pharmaceutical composition according to any one of the preceding clauses, wherein the pharmaceutical composition is manufactured by dry mix techniques selected from dry granulation or direct compression, preferably direct compression.
25.- The pharmaceutical composition according to any one of the preceding clauses, wherein the pharmaceutical composition is manufactured wet granulation techniques.
26.- A pharmaceutical batch comprising at least 50,000 units of the pharmaceutical composition as defined in any one of clauses 1 to 25.
27.- The pharmaceutical batch according to any one of the preceding clause, comprising at least 100,000 units.
28.- The pharmaceutical batch according to any one of the two preceding clauses, comprising at least 500,000 units.
29.- The pharmaceutical batch according to any one of the three preceding clauses, wherein the content of the active agent is uniform.
30.- The pharmaceutical batch according to any one of the four preceding clauses, wherein the pharmaceutical compositions are packaged in a blister pack of aluminum/PVC, aluminum/aluminum or a perforated aluminium/PVC/polyvinyl acetate copolymer-acrylate blister.
31.- A process for preparing a pharmaceutical composition as defined in clauses 1 to 25 or the pharmaceutical batch as defined in clauses 26 to 31, wherein the process comprises the following steps:
   (i) weighing, optionally sieving and mixing the active agent and optionally at least one pharmaceutically acceptable excipient,
   (ii) blending the mix of step (i),
   (iii) optionally granulating the blend
   (iv) optionally drying the granulate obtained in step (ii),
   (v) compressing the blend obtained in step (ii) or the granules obtained in step (iii) or (iv) to form a tablet, and
   (vi) coating the tablet composition.
32.- The pharmaceutical composition or the pharmaceutical batch obtained by the process according to the preceding clauses.
33.- The pharmaceutical composition according to any one of clauses 1 to 23 or 32 or the pharmaceutical batch according to any one of clauses 26 to 30 or 32, for use in the treatment of type 2 diabetes mellitus alone or in combination with other antidiabetic agents.
34.- A blister pack comprising the pharmaceutical composition as defined in any one of clauses 1 to 23 or 32 or the pharmaceutical batch according to any one of clauses 26 to 30 or 32, wherein said blister pack is preferably an aluminum/PVC blister, an aluminum/aluminum blister, or a perforated aluminium/PVC/polyvinyl acetate copolymer-acrylate blister.
35.- A cardboard box with a patient information leaflet comprising at least one aluminium/aluminium or aluminium/PVC blister pack of at least 4 units of the pharmaceutical composition as defined in any one of the clauses 1 to 23 or 32 or or the pharmaceutical batch according to any one of clauses 26 to 30 or 32.
36.- A method for preparing a pharmaceutical dossier to obtain the marketing authorization of pharmaceutical composition as defined in any one of clauses 1 to 23 or 32 comprising the following steps:
   i) manufacturing at least one pharmaceutical batch as defined in any one of clauses 26 to 30 or 32;
   ii) performing stability tests of the batches of step (i);
   iii) compiling the results obtained in steps (i) to (iii); and
   iv) providing the compiled results of step (iii) in a data carrier.
37.- A data carrier comprising the compiled results of a pharmaceutical dossier obtained by the method of the preceding clause.
38.- The data carrier according to the preceding clause, wherein said data carrier is selected from the group consisting of a digital data carrier such as CD, DVD, USB, hard drive; and paper.

### EXAMPLES

Example 1: 5 mg linagliptin tablet composition manufactured by wet granulation

| Ingredient | % w/w* |
|---|---|
| *Tablet core* | |
| Linagliptin | 2 - 5 % |
| Trehalose | 65 - 85 % |
| Sodium starch glycolate | 5 - 10 % |
| Copovidone | 2 - 6 % |
| Magnesium stearate | 1 - 3 % |
| *Film coating* | |
| Hydroxypropylmethylcelullose | 0.5 - 2.5 % |
| Titanium dioxide (E171) | 0.01 - 1.5 % |
| Talc | 0.05 - 1 % |
| Macrogol (6000) | 0.05 - 1 % |
| Iron oxide red (E172) | 0.01 - 0.2 % |

| | |
|---|---|
| * in respect of the total weight of the composition. | |

Copovidone is dissolved in purified water at ambient temperature to produce a granulation liquid.

Linagliptin and trehalose are blended in a suitable mixer to produce the pre-mix. The pre-mix is moistened with the granulation liquid and subsequently granulated using a high shear mixer. The moist granulate is optionally sieved through a sieve with a mesh size of 1.6 - 3.0 mm. The granulate is dried at about 40 °C in a fluid bed dryer until a loss on drying value of 1.5 - 4 % is obtained.

The dry granulate obtained is sifted and mixed with the sodium starch glycolate and then, lubricated with the magnesium stearate. The final blend is compressed into tablet cores.

Hydroxypropyl methylcellulose, polyethylene glycol, talc, titanium dioxide and iron oxide (or an equivalent ready-to-use product) are suspended in purified water in a suitable mixer at ambient temperature to produce a coating suspension. The tablet cores are coated with the coating suspension to a weight gain of about 2 - 3 % w/w in respect of the total weight of the composition to produce film-coated tablets.

### Example 2:5 mg linagliptin tablet composition

| Ingredient | % w/w* |
|---|---|
| *Tablet core* | |
| Linagliptin | 2 - 5 % |
| Trehalose | 25 - 45 % |
| Sorbitol | 40 - 60 % |
| Sodium starch glycolate | 4 - 10 % |
| Copovidone | 2 - 6 % |
| Magnesium stearate | 1 - 3 % |
| *Film coating* | |
| Hydroxypropylmethylcelullose | 0.5 - 2.5 % |
| Titanium dioxide (E171) | 0.015 - 1.5 % |
| Talc | 0.055 - 1 % |
| Macrogol (6000) | 0.05 - 10.5 % |
| Iron oxide red (E172) | 0.015 - 0.2 % |

| | |
|---|---|
| * in respect of the total weight of the composition. | |

Copovidone is dissolved in purified water at ambient temperature to produce a granulation liquid.

Linagliptin, trehalose and sorbitol are blended in a suitable mixer to produce the pre-mix. The pre-mix is moistened with the granulation liquid and subsequently granulated using a high shear mixer. The moist granulate is optionally sieved through a sieve with a mesh size of 1.6 - 3.0 mm. The granulate is dried at about 40 °C in a fluid bed dryer until a loss on drying value of 1.5 - 4 % is obtained.

The dry granulate obtained is sifted and mixed with the sodium starch glycolate and then, lubricated with the magnesium stearate. The final blend is compressed into tablet cores.

Hydroxypropyl methylcellulose, polyethylene glycol, talc, titanium dioxide and iron oxide (or an equivalent ready-to use product) are suspended in purified water in a suitable mixer at ambient temperature to produce a coating suspension. The tablet cores are coated with the coating suspension to a weight gain of about 2 - 3 % to produce film-coated tablets.

### Example 3:5 mg linagliptin tablet composition by direct compression

| Ingredient | % w/w* |
|---|---|
| *Tablet core* | |
| Linagliptin | 2-5% |
| Microcrystalline cellulose | 45 - 65 % |
| Lactose anhydrous | 25 - 40 % |
| Sodium starch glycolate | 4 - 10% |
| Copovidone | 2 - 6% |
| Magnesium stearate | 1 - 3% |
| *Film coating* | |
| Hydroxypropylmethylcelullose | 0.5 - 2.5 % |
| Titanium dioxide (E171) | 0.015 - 1.5 % |
| Talc | 0.055 - 1 % |
| Macrogol (6000) | 0.05 - 10.5 % |
| Iron oxide red (E172) | 0.015 - 0.2 % |

| | |
|---|---|
| * in respect of the total weight of the composition. | |

Linagliptin, lactose anhydrous and copovidone are mixed in a suitable blender. Then microcrystalline cellulose and sodium starch glycolate are added into the blender and mixed; finally, magnesium stearate is added into the blender in order to obtain the final blend. The final blend is compressed into tablet cores.

Hydroxypropyl methylcellulose, polyethylene glycol, talc, titanium dioxide and iron oxide (or an equivalent ready-to use product) are suspended in purified water in a suitable mixer at ambient temperature to produce a coating suspension. The tablet cores are coated with the coating suspension to a weight gain of about 2 - 3 % to produce film-coated tablets.

### Example 4: Synthesis of Linagliptin

The process for the synthesis of linagliptin mixture of forms A and B is described in WO2004018468.

### Examples 5: Particle size volume distribution

The particle size distribution of linagliptin was analysed by laser diffraction spectroscopy using a Malvern Mastersizer 2000 particle size analyzer. The particle size distribution has a D90 of less than 800 microns, preferably of less than 500 microns.

## Claims

1. A stable pharmaceutical composition of linagliptin in the form of immediate release tablets comprising a mixture of linagliptin crystalline forms A and B and a pharmaceutically acceptable excipient, wherein:
i) the total amount of active ingredient present in the pharmaceutical composition ranges from 1 to 10 % w/w, preferably ranges from 2 to 5% w/w; and the total amount of diluent or mixture of diluents present in the pharmaceutical composition is at least 60 % w/w, preferably ranges from 65 to 90% w/w; both in respect of the total amount of the pharmaceutical composition, and
ii) the tablet consists of a core and a coating, wherein the core consists of compressed particles wherein the D90 of such particles is less than 800 microns, preferably the the D90 of the particles to be compressed is less than 500 microns, preferably the Carr's index of the particles to be compressed is less than 15,
preferably wherein the mixture of crystalline form linagliptin A and B used for the composition has a melting temperature of 198-202 °C.

2. The pharmaceutical composition according to any one of the preceding claims, wherein the weight ratio linagliptin form A:form B ranges from 99:1 to 1:99, preferably the weight ratio linagliptin form A:form B ranges from 55:45 to 65:35.

3. The pharmaceutical composition according to any one of the preceding claims, wherein the total content of impurities originating from the decomposition of linagliptin is less than 10 % w/w in respect of the total amount of the pharmaceutical composition after storage for 2 months at 40 °C at 75% RH, preferably the total content of impurities originating from the decomposition of linagliptin is less than 3 % w/w in respect of the total amount of the pharmaceutical composition after storage for 2 months at 40 °C at 75% RH and/or wherein the total content of impurities originating from the decomposition of linagliptin is less than 10 % w/w in respect of the total amount of the pharmaceutical composition after storage for 2 months at 25°C at 60% RH, preferably the total content of impurities originating from the decomposition of linagliptin is less than 3 % w/w in respect of the total amount of the pharmaceutical composition for 2 months at 25°C at 60% RH.

4. The pharmaceutical composition according to any one of the preceding claims, wherein the mixture of linagliptin crystalline forms A and B does not change into any other crystalline form.

5. The pharmaceutical composition according to any one of the preceding claims, wherein the pharmaceutical composition is free of mannitol, preferably the pharmaceutical composition comprises trehalose, pregelatinized starch or mixtures thereof; and/or the pharmaceutical composition comprises only one diluent, preferably the pharmaceutical composition comprises trehalose or pregelatinized starch; or the pharmaceutical composition comprises at least two diluents different than mannitol and pregelatinized starch, preferably the pharmaceutical composition comprises trehalose, sorbitol or mixtures thereof.

6. The pharmaceutical composition according to any one of the preceding claims, wherein the pharmaceutical composition comprises at least a disintegrant, preferably the total amount of disintegrant or disintegrants present in the pharmaceutical composition ranges from 1 to 15 % w/w in respect of the total amount of the pharmaceutical composition; and/or the pharmaceutical composition comprises at least a disintegrant selected from croscarmellose sodium, crospovidone, sodium glycolate or mixtures thereof; and/or the pharmaceutical composition comprises at least a binder, preferably the total amount of binder or binders present in the pharmaceutical composition ranges from 1 to 8 % w/w in respect of the total amount of the pharmaceutical composition; and/or the pharmaceutical composition comprises copovidone; and/or the pharmaceutical composition comprises at least a lubricant, preferably the total amount of lubricant or lubricants present in the pharmaceutical composition ranges from 0.5 to 5 % w/w in respect of the total amount of the pharmaceutical composition; and/or the pharmaceutical composition comprises magnesium stearate.

7. The pharmaceutical composition according to any one of the preceding claims, wherein the pharmaceutical composition is manufactured by dry mix techniques or wet granulation techniques, preferably the dry mix techniques are selected from dry granulation or direct compression, more preferably direct compression.

8. A pharmaceutical batch comprising at least 50,000 units of the pharmaceutical composition as defined in any one of claims 1 to 7, preferably the batch comprises at least 500,000 units, more preferably the content of the active agent in the batch is uniform, preferably the pharmaceutical compositions are packaged in a blister pack of aluminum/PVC, aluminum/aluminum or a perforated aluminium/PVC/polyvinyl acetate copolymer-acrylate blister.

9. A process for preparing a pharmaceutical composition as defined in claims 1 to 7 or the pharmaceutical batch as defined in claim 8, wherein the process comprises the following steps:
(i) weighing, optionally sieving and mixing the active agent and optionally at least one pharmaceutically acceptable excipient,
(ii) blending the mix of step (i),
(iii) optionally granulating the blend
(iv) optionally drying the granulate obtained in step (ii),
(v) compressing the blend obtained in step (ii) or the granules obtained in step (iii) or (iv) to form a tablet, and
(vi) coating the tablet composition.

10. The pharmaceutical composition or the pharmaceutical batch obtained by the process according to the preceding claim.

11. The pharmaceutical composition according to any one of claims 1 to 7 or 10 or the pharmaceutical batch according to any one of claims 8 or 10, for use in the treatment of type 2 diabetes mellitus alone or in combination with other antidiabetic agents.

12. A blister pack comprising the pharmaceutical composition as defined in any one of claims 1 to 7 or 10 or the pharmaceutical batch according to any one of claims 8 or 10, wherein said blister pack is preferably an aluminum/PVC blister, an aluminum/aluminum blister, or a perforated aluminium/PVC/polyvinyl acetate copolymer-acrylate blister.

13. A cardboard box with a patient information leaflet comprising at least one aluminium/aluminium or aluminium/PVC blister pack of at least 4 units of the pharmaceutical composition as defined in any one of the claims 1 to 7 or 10 or the pharmaceutical batch according to any one of claims 8 or 10.

14. A method for preparing a pharmaceutical dossier to obtain the marketing authorization of pharmaceutical composition as defined in any one of claims 1 to 7 or 10 comprising the following steps:
i) manufacturing at least one pharmaceutical batch as defined in any one of claims 8 or 10;
ii) performing stability tests of the batches of step (i);
iii) compiling the results obtained in steps (i) to (iii); and
iv) providing the compiled results of step (iii) in a data carrier.

15. A data carrier comprising the compiled results of a pharmaceutical dossier obtained by the method of the preceding claim, preferably said data carrier is selected from the group consisting of a digital data carrier such as CD, DVD, USB, hard drive; and paper.
